# EUROPEAN PATENT APPLICATION

(11) **EP 3 130 304 A1**
(43) Date of publication of application: **15.02.2017**
(21) Application number: 15776442.4
(22) Date of filing: 12.02.2015
(51) Int. Cl.: A61B 18/12, A61B 17/28, A61B 90/00

(54) **TREATMENT TOOL AND SURGICAL SYSTEM**

(30) Priority: 09.04.2014 JP 2014080193
(71) Applicant: Olympus Corporation, Hachioji-shi, Tokyo 192-8507 (JP)
(72) Inventor: KIKUCHI, Chisato, Hachioji-shi, Tokyo 192-8507 (JP)
(74) Representative: Schicker, Silvia
(86) International application number: PCT/JP2015/053852
(87) International publication number: WO 2015/156026

(57) **Abstract**

A treatment device (1) according to the present invention includes a narrow main unit, an end effector (3), a rolling joint (8) provided between the end effector (3) and the main unit, a driven joint (7) that is provided at the end effector (3) or between the end effector (3) and the rolling joint (8) and that rotates together with the rolling joint (8) about a longitudinal axis (A), a plurality of power transmission members (10A, 10B) that extend parallel to each other from the driven joint (7) to the main unit, a position maintaining means (13, 14) that that maintains the power transmission members (10A, 10B) at two positions located between the driven joint (7) and the main unit and separated from each other by a certain distance in a direction of the longitudinal axis (A), and a shaft member (12) disposed along the longitudinal axis (A) between the two positions. The position maintaining means (13, 14) maintains the power transmission members (10A, 10B) at predetermined relative positions separated from each other by the certain distance in a circumferential direction of the shaft member (12).

## Description

### {Technical Field}

The present invention relates to treatment devices and surgical systems.

### {Background Art}

As robotic surgeries have been becoming more widespread in recent years, robotic treatment devices are being used more and more in abdominal and thoracic surgeries and intraluminal surgeries (for example, see Patent Literature 1). A robotic treatment device according to Patent Literature 1 includes, in the following order from the proximal end thereof, a narrow shaft, a rolling joint, a bendable joint, and an end effector. In this articulated structure, rotational movement of the end effector can be performed instinctively by means of the rolling joint that moves in a manner similar to a human wrist.

### {Citation List}

### {Patent Literature}

{PTL 1}
US Patent No. 6746443

### {Summary of Invention}

### {Technical Problem}

However, in the articulated structure according to Patent Literature 1, cables used for mechanically driving the end effector and the bendable joint by transmitting power thereto extend to the shaft via the rolling joint. When the rolling joint rotates in this structure, the twisted cables come into contact with each other, causing friction to occur between the cables. This friction inhibits the transmission of the power to the bendable joint and the end effector. This is problematic in terms of reduced responsiveness of the end effector and the bendable joint to a manipulation.

The present invention has been made in view of the circumstances described above, and an object thereof is to provide a treatment device and a surgical system, in which the treatment device employs an articulated structure having a rolling joint and a driven joint provided toward the distal end relative to the rolling joint and can maintain the responsiveness of the driven joint to a manipulation even when the rolling joint is rotated.

### {Solution to Problem}

In order to achieve the aforementioned object, the present invention provides the following solutions.

A first aspect of the present invention provides a treatment device including a narrow main unit; an end effector located at a distal end of the main unit; a rolling joint that is provided between the end effector and the main unit and that is rotatable relative to the main unit about a longitudinal axis of the main unit; a driven joint that is provided at the end effector or between the end effector and the rolling joint and that rotates together with the rolling joint about the longitudinal axis; a plurality of linear power transmission members that extend parallel to each other from the driven joint to the main unit via the rolling joint and that transmit power applied to proximal ends; a position maintaining means that maintains the plurality of power transmission members at predetermined relative positions, which are two positions located between the driven joint and the main unit and separated from each other by a certain distance in a direction of the longitudinal axis; and a columnar shaft member disposed in the direction of the longitudinal axis between the two positions. The position maintaining means maintains the plurality of power transmission members at the relative positions located at a radially outer side of the shaft member and separated from each other by the certain distance in a circumferential direction of the shaft member.

According to the first aspect of the present invention, the main unit is inserted into a body, starting from the end effector, and the end effector is moved inside the body by driving the rolling joint and the driven joint, so that the end effector can be positioned relative to an affected area to treat the affected area. Specifically, by rotating the rolling joint, the end effector and the driven joint located toward the distal end relative to the rolling joint can be rotated. Furthermore, the driven joint is driven by supplying power to the driven joint via the power transmission members, so as to cause the end effector to perform predetermined movement.

In this case, when the rolling joint rotates, the distal ends of the power transmission members connected to the driven joint rotate together with the driven joint, whereas the proximal ends of the power transmission members located within the main unit do not rotate. Therefore, the power transmission members become twisted around the outer peripheral surface of the shaft member between the driven joint and the main unit.

In this case, at the distal end and the proximal end of the shaft member, the relative positions of the plurality of power transmission members are maintained by the position maintaining means at positions separated from each other by a certain distance in the circumferential direction of the shaft member. Therefore, the power transmission members do not come into contact with each other even in the twisted state, so that the power applied to the proximal ends of the power transmission members is transmitted to the driven joint with high efficiency. Consequently, the responsiveness of the driven joint to a manipulation can be maintained even when the rolling joint is rotated.

In the first aspect, the shaft member may be provided in a rotatable manner in the circumferential direction relative to the driven joint.

Accordingly, when the power transmission members come into contact with the outer peripheral surface of the shaft member, the shaft member rotates in accordance with the force received by the power transmission members, so that a frictional force between the shaft member and the power transmission members is reduced. Consequently, the power transmission efficiency of the power transmission members is further improved, thereby further enhancing the responsiveness of the driven joint.

In the first aspect, the treatment device may further include a small-diameter extension member whose distal end is connected to the end effector and that extends from the end effector to the main unit. The shaft member may have a hole extending therethrough in the direction of the longitudinal axis and accommodating a longitudinal section of the extension member.

The end effector, such as an energy device, grasping forceps, a local injection needle, a suction tube, or a clip, is provided with the extension member, such as an electric wire or a pipe line, for allowing the end effector to function. By accommodating such an extension member inside the shaft member, the extension member can be protected from the twisted power transmission members, so that the function of the end effector can be properly maintained.

In the first aspect, the shaft member may be fixed to an outer peripheral surface of the extension member.

Accordingly, by combining the longitudinal section of the extension member with the shaft member, the number of components can be reduced, thereby facilitating the assembly process.

In the first aspect, the shaft member may have a groove that extends in the circumferential direction and that is located at a position where an outer peripheral surface of the shaft member comes into contact with the power transmission members when the rolling joint rotates.

Accordingly, the power transmission members twisted as a result of rotation of the rolling joint fit into the groove so as to be temporarily fixed to the outer peripheral surface of the shaft member. This can more reliably prevent the power transmission members from coming into contact with each other.

A second aspect of the present invention provides a treatment device including a narrow main unit, an end effector located at a distal end of the main unit, a rolling joint that is provided between the end effector and the main unit and that is rotatable relative to the main unit about a longitudinal axis of the main unit, a driven joint that is provided at the end effector or between the end effector and the rolling joint and that rotates together with the rolling joint about the longitudinal axis, a plurality of linear power transmission members that extend parallel to each other from the driven joint to the main unit via the rolling joint and that transmit power applied to proximal ends, and a tubular sheath that is composed of an elastic material and that covers outer peripheral surfaces of the plurality of power transmission members between the driven joint and the main unit.

According to the second aspect of the present invention, when the rolling joint rotates, the power transmission members become twisted between the driven joint and the main unit. In this case, since the power transmission members are protected by the sheath, the power transmission members do not come into contact with each other even in the twisted state, so that the power applied to the proximal ends of the power transmission members is transmitted to the driven joint with high efficiency. Consequently, the responsiveness of the driven joint to a manipulation can be maintained even when the rolling joint is rotated.

In the second aspect, the sheath may be formed of a single component having a plurality of lumens extending therethrough in a longitudinal direction, and the power transmission members may be respectively inserted through the plurality of lumens.

Accordingly, the number of components can be reduced.

In the second aspect, a plurality of the sheaths may be provided individually for the respective power transmission members.

Accordingly, since the amount of twisting occurring in the sheaths as a result of twisting of the power transmission members can be reduced, the elasticity required in each sheath can be reduced, whereby a material with low elasticity can be used for each sheath.

A third aspect of the present invention provides a surgical system including the treatment device according to one of the above aspects, a manipulation input device that is to be manipulated by an operator, a driving device that drives at least one of the rolling joint and the driven joint, and a control device that controls the driving device on the basis of a manipulation input to the manipulation input device.

According to the third aspect of the present invention, the driving device is configured to be electrically controlled by the control device, so that the rolling joint and/or the driven joint can be manipulated from a location distant from the treatment device via the manipulation input device provided separately from the treatment device.

### {Advantageous Effects of Invention}

The present invention is advantageous in that it can maintain the responsiveness of the driven joint to a manipulation even when the rolling joint is rotated.

### {Brief Description of Drawings}

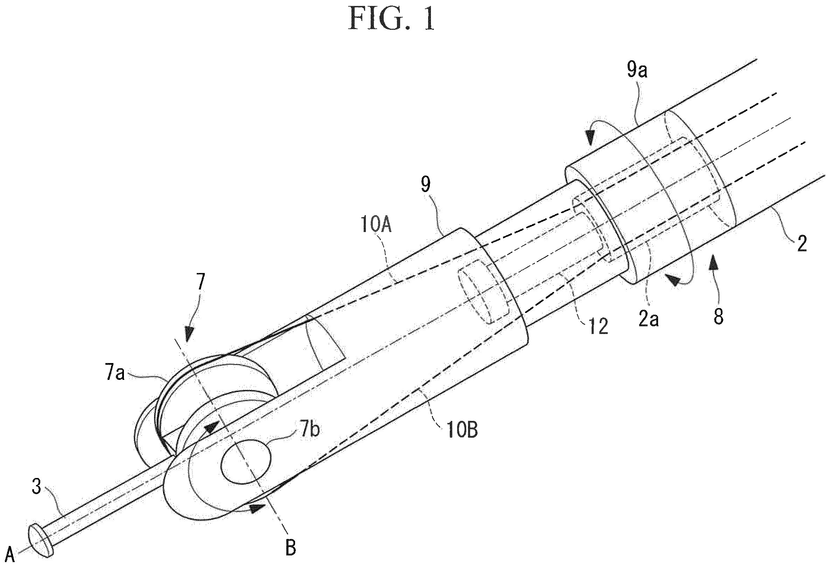

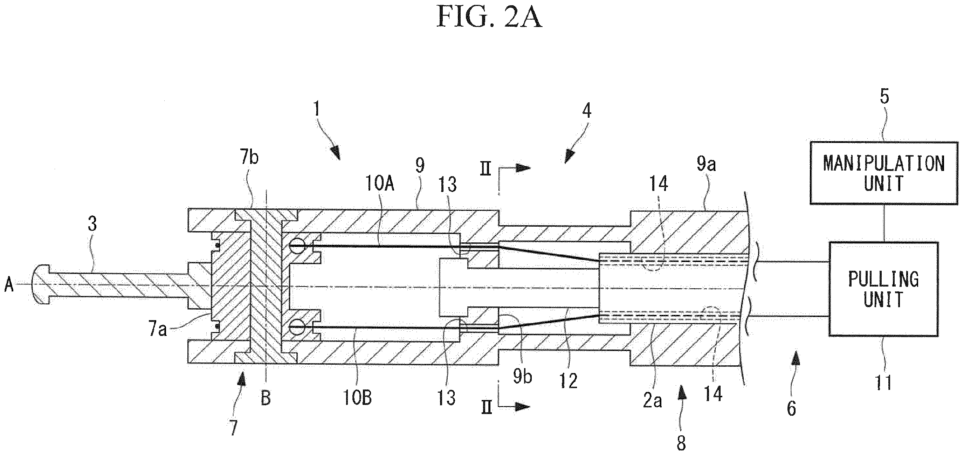

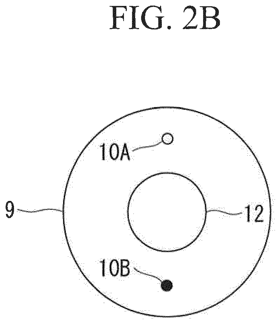

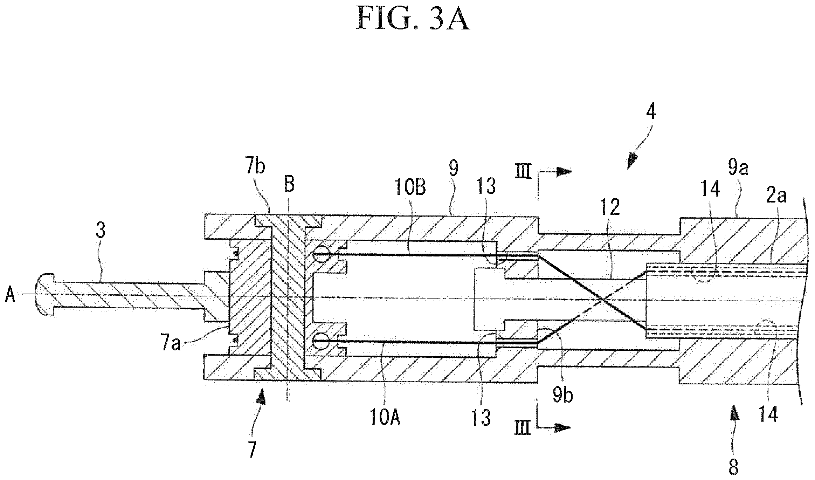

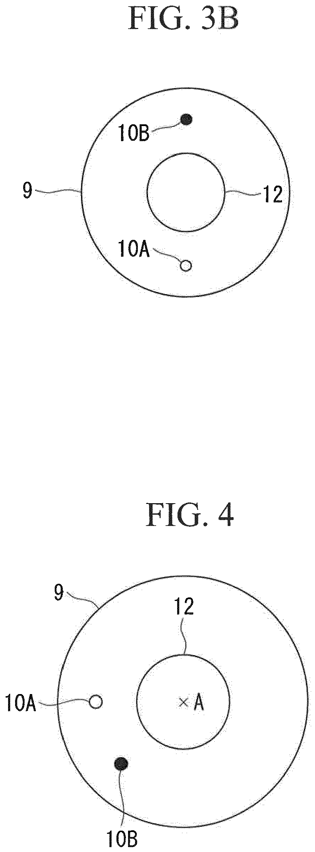

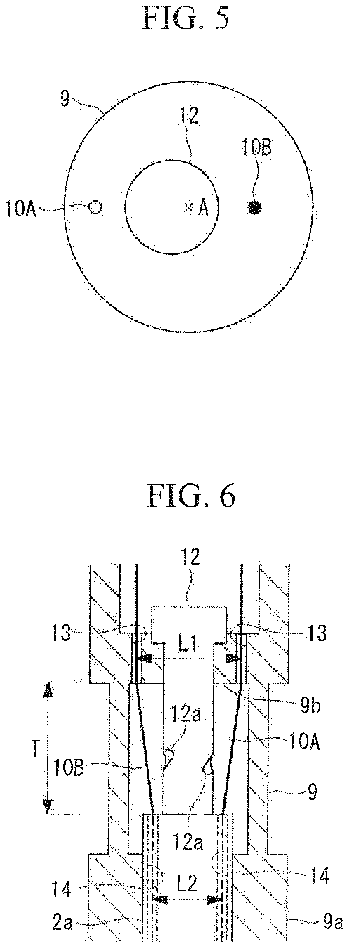

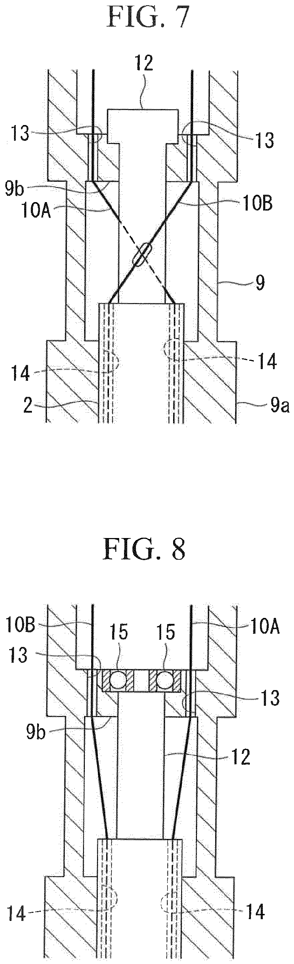

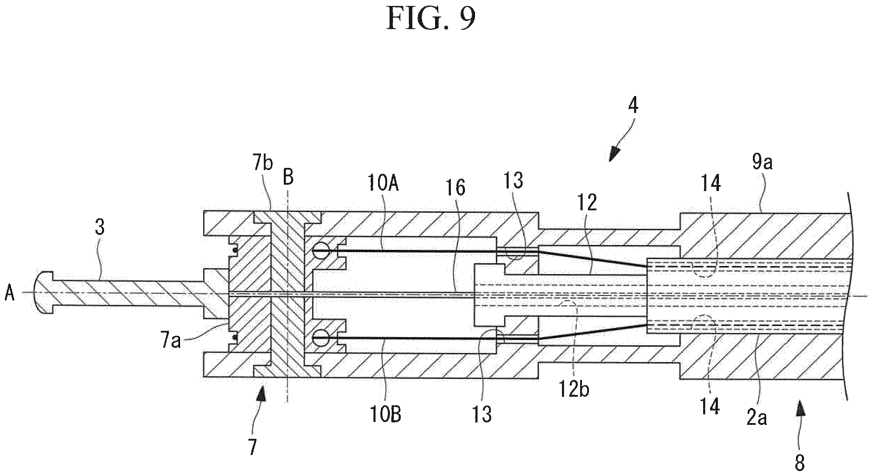

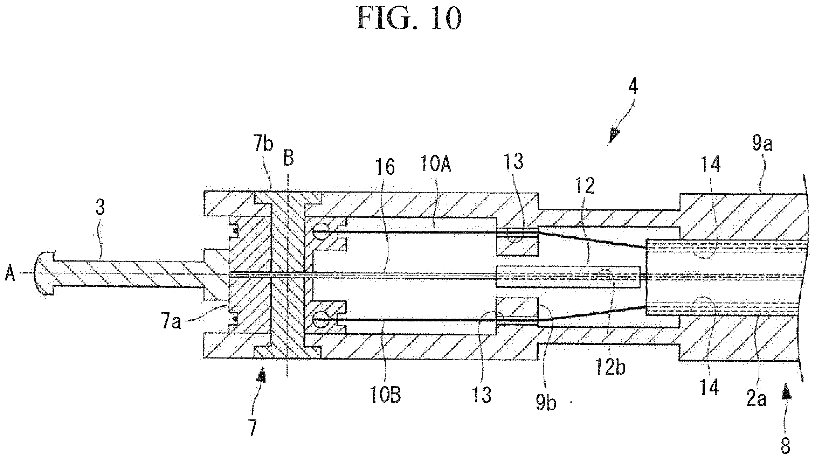

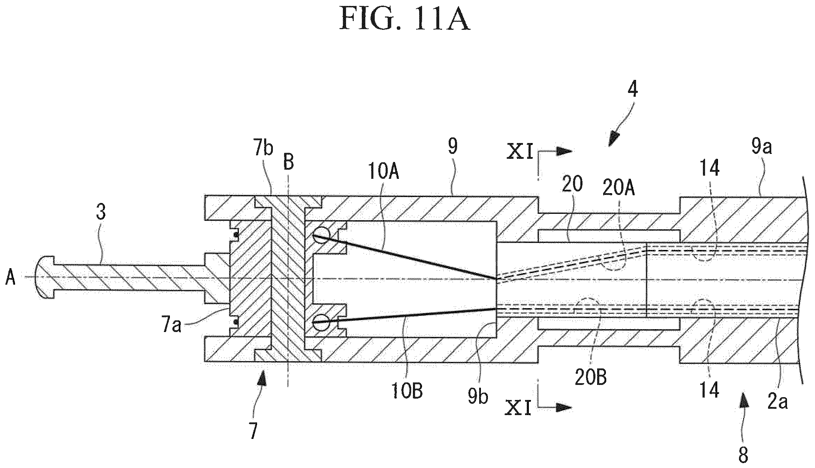

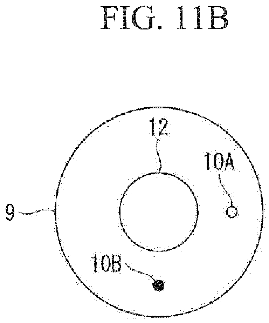

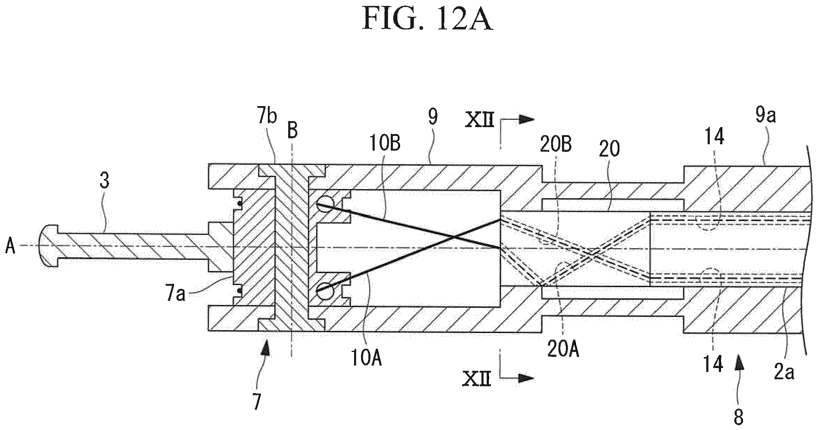

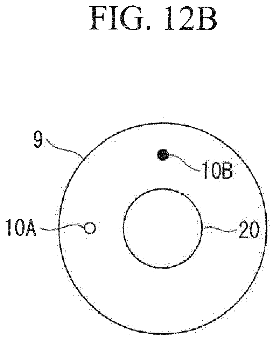

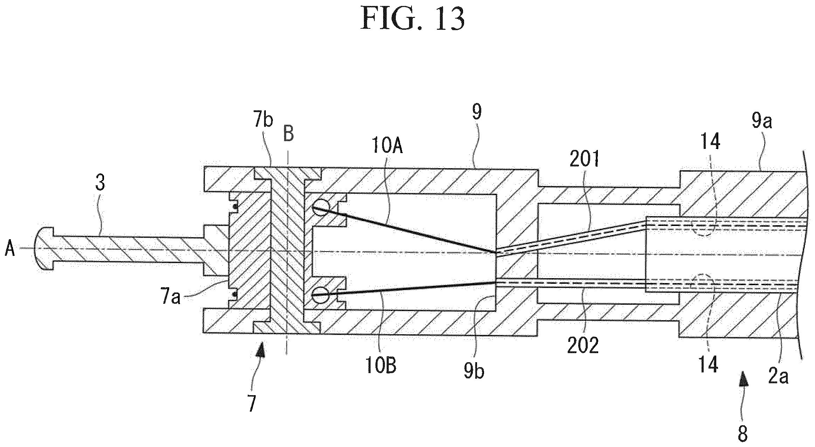

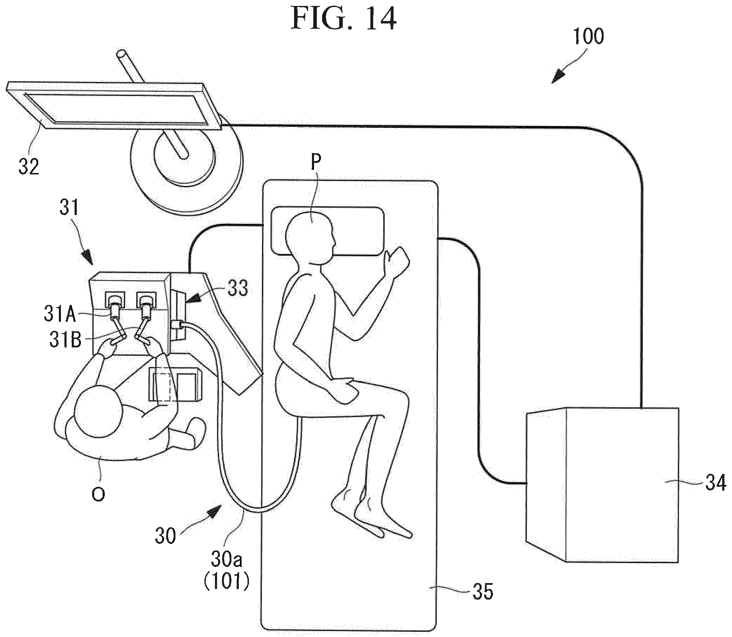

{Fig. 1} Fig. 1 is a perspective view of a distal end of a treatment device according to a first embodiment of the present invention.
{Fig. 2A} Fig. 2A illustrates the overall configuration of the treatment device in Fig. 1 and the internal structure of the distal end of the treatment device.
{Fig. 2B} Fig. 2B is a simplified cross-sectional view taken along line II-II in Fig. 2A.
{Fig. 3A} Fig. 3A illustrates the internal structure of the distal end of the treatment device in Fig. 1 when a rolling joint is rotated by 180°.
{Fig. 3B} Fig. 3B is a simplified cross-sectional view taken along line III-III in Fig. 3A.
{Fig. 4} Fig. 4 illustrates a modification of the relative positions of two wires held by wire holders.
{Fig. 5} Fig. 5 illustrates another modification of the relative positions of the two wires held by the wire holders.
{Fig. 6} Fig. 6 illustrates the internal structure of the distal end according to a modification of the treatment device in Fig. 1.
{Fig. 7} Fig. 7 illustrates the internal structure of the distal end of the treatment device in Fig. 6 when the rolling joint is rotated by 180°.
{Fig. 8} Fig. 8 illustrates the internal structure of the distal end according to another modification of the treatment device in Fig. 1.
{Fig. 9} Fig. 9 illustrates the internal structure of the distal end according to another modification of the treatment device in Fig. 1.
{Fig. 10} Fig. 10 illustrates the internal structure of the distal end according to another modification of the treatment device in Fig. 1.
{Fig. 11A} Fig. 11A illustrates the internal structure of the distal end of a treatment device according to a second embodiment of the present invention.
{Fig. 11B} Fig. 11B is a simplified cross-sectional view taken along line XI-XI in Fig. 11A.
{Fig. 12A} Fig. 12A illustrates the internal structure of the distal end of the treatment device in Fig. 11A when the rolling joint is rotated by 180°.
{Fig. 12B} Fig. 12B is a simplified cross-sectional view taken along line XII-XII in Fig. 12A.
{Fig. 13} Fig. 13 illustrates the internal structure of the distal end according to a modification of the treatment device in Fig. 11A.
{Fig. 14} Fig. 14 illustrates the overall configuration of a surgical system according to a third embodiment of the present invention.

### {Description of Embodiments}

### {First Embodiment}

A treatment device 1 according to a first embodiment of the present invention will be described below with reference to Figs. 1 to 10.

As shown in Figs. 1, 2A, and 2B, the treatment device 1 according to this embodiment includes a narrow main unit 2 that is insertable into a body, an end effector 3 located at the distal end of the main unit 2, a joint unit 4 provided between the main unit 2 and the end effector 3, a manipulation unit 5 provided at the proximal end of the main unit 2, and a driving mechanism 6 that drives the joint unit 4 in accordance with a manipulation input to the manipulation unit 5.

The end effector 3 is, for example, an energy device, grasping forceps, a local injection needle, a suction tube, or a clip. The end effector 3 shown in the reference drawings is, for example, a high-frequency energy device.

The joint unit 4 has a bendable joint 7 and a rolling joint 8 in that order from the distal end thereof. Reference sign 9 denotes a substantially cylindrical housing that accommodates the joint unit 4.

The bendable joint 7 is provided in a bendable manner about an axis B that is orthogonal to a longitudinal axis A of the main unit 2. Specifically, the bendable joint 7 is constituted of a pulley 7a and a rotating shaft 7b with which the pulley 7a is attached to the housing 9 in a rotatable manner about the axis B. The end effector 3 is fixed to the pulley 7a such that, when the bendable joint 7 bends as a result of rotation of the pulley 7a, the end effector 3 swivels about the axis B.

The rolling joint 8 is provided in a rotatable manner about the longitudinal axis A relative to the main unit 2. Specifically, the rolling joint 8 is formed by fitting a proximal end 9a of the housing 9 around a shaft 2a, which is formed of the distal end of the main unit 2, in a rotatable manner in the circumferential direction. When the rolling joint 8 rotates as a result of the proximal end 9a rotating around the shaft 2a, the bendable joint 7 supported by the housing 9 and the end effector 3 fixed to the bendable joint 7 also rotate about the longitudinal axis A together with the rolling joint 8. Figs. 2A and 2B illustrate a state where the rotational angle of the rolling joint 8 is 0°.

The manipulation unit 5 has a component, such as a dial or a joystick, to which a predetermined motion can be input.

The driving mechanism 6 includes two wires (power transmission members) 10A and 10B extending parallel to each other from the pulley 7a to the main unit 2 via the joint unit 4 and also includes a pulling unit 11 that pulls the proximal ends of the wires 10A and 10B in the direction of the longitudinal axis A in conjunction with the motion input to the manipulation unit 5.

The two wires 10A and 10B have their distal ends fixed to the pulley 7a and are wound around the pulley 7a in opposite directions from each other from their respective fixation positions. Thus, the rotational direction of the pulley 7a when one of the two wires 10A and 10B is pulled and the rotational direction of the pulley 7a when the other wire is pulled are opposite from each other, so that the end effector 3 can be swiveled about the axis B in both directions.

The pulling unit 11 is constituted of, for example, a combination of gears and converts the motion input to the manipulation unit 5 into a motion in the direction of the longitudinal axis A so as to pull the proximal end of one of the wires 10A and 10B. In this case, in synchronization with the pulling of one of the wires 10A and 10B, it is preferable that the other wire be fed. The pulling motion in the direction of the longitudinal axis A applied to the proximal end of the wire 10A or 10B from the pulling unit 11 is transmitted to the distal end of the wire 10A or 10B and is further transmitted to the pulley 7a. Thus, the pulley 7a rotates clockwise or counterclockwise about the axis B, thereby causing the bendable joint 7 to bend.

The rolling joint 8 also receives the motion input to the manipulation unit 5 via a power transmission member, such as a wire (not shown), so as to be rotationally driven.

Furthermore, the treatment device 1 according to this embodiment includes a shaft member 12 disposed between the bendable joint 7 and the main unit 2 in the direction of the longitudinal axis A and also includes wire holders (position maintaining means) 13 and 14 that maintain the intermediate positions of the two wires 10A and 10B in the longitudinal direction at predetermined relative positions at the distal end and the proximal end of the shaft member 12.

The shaft member 12 is a rigid columnar component composed of, for example, polyether ether ketone or stainless steel. The distal end of the shaft member 12 is fixed to a ring-shaped inner wall 9b protruding radially inward from the inner peripheral surface of the housing 9, so that that the shaft member 12 also rotates together with the rolling joint 8 about the longitudinal axis A.

The wire holders are constituted of two small-diameter through-holes 13 extending through the inner wall 9b in the direction of the longitudinal axis A and two small-diameter channels 14 extending from the distal-end surface of the shaft 2a toward the proximal end thereof in the direction of the longitudinal axis A. The two through-holes 13 are provided in the inner wall 9b at positions separated from each other by 180° in the circumferential direction. The two wires 10A and 10B are inserted through the different through-holes 13 in a movable manner in the direction of the longitudinal axis A. The two channels 14 are provided at the radially outer side of the shaft member 12 at positions separated from each other by 180° in the circumferential direction. The two wires 10A and 10B are inserted through the different channels 14 in a movable manner in the direction of the longitudinal axis A. Accordingly, in the state where the rotational angle of the rolling joint 8 is 0°, the two wires 10A and 10B extending from the through-holes 13 to the distal end of the main unit 2 are arranged substantially parallel to each other at the radially outer side of the shaft member 12.

Next, the operation of the treatment device 1 having the above-described configuration will be described.

In the treatment device 1 according to this embodiment, when a surgeon manipulates the manipulation unit 5, the driving mechanism 6 bends the bendable joint 7 or rotates the rolling joint 8 in accordance with the manipulation input to the manipulation unit 5. Accordingly, the position and orientation of the distal end of the end effector 3 inserted in the body can be changed via the manipulation unit 5 disposed outside the body.

The wires 10A and 10B have their distal ends fixed to the bendable joint 7 and their proximal ends held within the main unit 2. Therefore, when the bendable joint 7 rotates relative to the main unit 2 in accordance with rotation of the rolling joint 8, the wires 10A and 10B become twisted around the outer peripheral surface of the shaft member 12 between the bendable joint 7 and the main unit 2, as shown in Figs. 3A and 3B. Figs. 3A and 3B illustrate a state where the rotational angle of the rolling joint 8 is 180°.

However, in the through-holes 13 at the distal end of the shaft member 12, the two wires 10A and 10B are prevented from moving in the radial direction and the circumferential direction with respect to the longitudinal axis A by the inner walls of the through-holes 13, so that the relative positions of the two wires 10A and 10B are maintained without changing before and after the rotation of the rolling joint 8. Likewise, in the distal end surface of the main unit 2 at the proximal end of the shaft member 12, the two wires 10A and 10B are prevented from moving in the radial direction and the circumferential direction with respect to the longitudinal axis A by the inner walls of the channels 14, so that the relative positions of the two wires 10A and 10B are maintained without changing before and after the rotation of the rolling joint 8.

Specifically, even in the state where the two wires 10A and 10B are twisted, as in Figs. 3A and 3B, the two wires 10A and 10B are maintained at relative positions separated from each other by a certain distance in the lateral direction, so that the wires 10A and 10B do not come into contact with each other. Therefore, even when the rolling joint 8 rotates, a pulling force applied to the proximal ends of the wires 10A and 10B from the pulling unit 11 is transmitted to the pulley 7a of the bendable joint 7 with high efficiency without attenuating. This is advantageous in that good responsiveness of the bendable joint 7 to a manipulation input to the manipulation unit 5 by the surgeon can be achieved.

In this embodiment, the wire holders 13 and 14 hold the wires 10A and 10B at relative positions separated from each other by 180° in the circumferential direction of the shaft member 12. However, the relative positions of the two wires 10A and 10B are not limited to these positions so long as the positions are separated from each other by a certain distance in the circumferential direction of the shaft member 12. For example, as shown in Fig. 4, the two wires 10A and 10B may be held asymmetrically with respect to the longitudinal axis A. Furthermore, as shown in Fig. 5, the shaft member 12 may be disposed at a position decentered from the longitudinal axis A, and the two wires 10A and 10B may be held at lopsided positions with respect to the longitudinal axis A.

Furthermore, as shown in Fig. 6, in this embodiment, the outer peripheral surface of the shaft member 12 may be provided with grooves 12a that can accommodate the wires 10A and 10B therein. The grooves 12a are formed at least in the outer peripheral surface of the shaft member 12 at positions where the grooves 12a come into contact with the respective wires 10A and 10B when the rolling joint 8 rotates. With regard to the configuration shown in Fig. 6, assuming that the center distance between the through-holes 13 is defined as L1, the center distance between the channels 14 is defined as L2, and the distance between the through-holes 13 and the channels 14 in the direction of the longitudinal axis A is defined as T, the contact position between the wires 10A and 10B and the shaft member 12 is located distant from the distal end surface of the shaft 2a by T×L2/(L1 + L2) toward the distal end.

With this configuration, when the rolling joint 8 rotates, the wires 10A and 10B fit into the grooves 12a, as shown in Fig. 7, so that the position of each of the wires 10A and 10B relative to the shaft member 12 is temporarily fixed. Consequently, the wires 10A and 10B can be more reliably prevented from coming into contact with each other.

Although the shaft member 12 is fixed to the bendable joint 7 by means of the housing 9 in this embodiment, the shaft member 12 may alternatively be provided in a rotatable manner in the circumferential direction thereof relative to the bendable joint 7, as shown in Fig. 8. In the configuration shown in Fig. 8, the shaft member 12 is supported by the housing 9 in a rotatable manner by means of a bearing 15 provided between the outer peripheral surface of the shaft member 12 and the inner peripheral surface of the inner wall 9b.

With this configuration, when the wires 10A and 10B come into contact with the shaft member 12, the force from the wires 10A and 10B causes the shaft member 12 to rotate in the circumferential direction, so that a frictional force between the wires 10A and 10B and the shaft member 12 is reduced. Consequently, attenuation of the pulling force transmitted through the wires 10A and 10B can be further reduced, thereby further enhancing the responsiveness of the bendable joint 7.

Furthermore, in this embodiment, the shaft member 12 may have a hole 12b extending therethrough in the direction of the longitudinal axis A, as shown in Fig. 9, and an extension member 16 extending from the end effector 3 to the main unit 2 may be accommodated within the hole 12b. The extension member 16 is a small-diameter member functioning as part of the end effector 3 and extending from the end effector 3 to the main unit 2 via the joint unit 4. For example, if the end effector 3 is an energy device, an electric wire for supplying high-frequency current is necessary. If the end effector 3 is a local injection needle, a pipe line for supplying an injection solution is necessary. If the end effector 3 is grasping forceps, a driving wire for opening and closing the grasping forceps is necessary.

The extension member 16, such as an electric wire, a pipe line, or a driving wire, is accommodated inside the shaft member 12 at the position where twisting of the wires 10A and 10B occurs, so that the extension member 16 is protected from the wires 10A and 10B twisted as a result of the rotation of the rolling joint 8, whereby the function of the end effector 3 can be properly maintained.

As shown in Fig. 10, the shaft member 12 may be combined with the extension member 16 into a single unit by fixing the inner peripheral surface of the hole 12b to the outer peripheral surface of the extension member 16. With this configuration, the number of components can be reduced.

### {Second Embodiment}

Next, a treatment device according to a second embodiment of the present invention will be described with reference to Figs. 11A to 13.

As shown in Figs. 11A and 11B, the treatment device according to this embodiment differs from the treatment device 1 according to the first embodiment in being provided with a sheath 20, which accommodates the two wires 10A and 10B, in place of the shaft member 12. Therefore, the following description of this embodiment will mainly focus on the sheath 20. Components similar to those in the first embodiment will be given the same reference signs, and descriptions thereof will be omitted.

The sheath 20 is a columnar component composed of an elastic material, such as silicone or vinyl chloride, and has two lumens 20A and 20B extending therethrough from the distal end surface to the proximal end surface. The lumens 20A and 20B have the wires 10A and 10B respectively inserted therethrough. The distal end and the proximal end of the sheath 20 are fixed to the inner wall 9b and the shaft 2a, respectively. Figs. 11A and 11B illustrate a state where the rotational angle of the rolling joint 8 is 0°.

Next, the operation of the treatment device having the above-described configuration will be described.

In the treatment device according to this embodiment, since the distal end and the proximal end of the sheath 20 are fixed to the bendable joint 7 and the main unit 2, respectively, when the bendable joint 7 rotates relative to the main unit 2 in accordance with rotation of the rolling joint 8, the sheath 20 becomes entirely twisted, as shown in Figs. 12A and 12B, thus also causing the wires 10A and 10B accommodated within the sheath 20 to twist. Figs. 12A and 12B illustrate a state where the rotational angle of the rolling joint 8 is 180°.

The two wires 10A and 10B, which are respectively accommodated within the different lumens 20A and 20B in the sheath 20 and whose outer peripheral surfaces are covered by the sheath 20, do not come into contact with each other even in the twisted state. Therefore, even when the rolling joint 8 rotates, a pulling force applied to the proximal ends of the wires 10A and 10B from the pulling unit 11 is transmitted to the pulley 7a of the bendable joint 7 with high efficiency without attenuating. This is advantageous in that good responsiveness of the bendable joint 7 to a manipulation input to the manipulation unit 5 by the surgeon can be achieved.

As an alternative to this embodiment in which a single sheath 20 having two lumens 20A and 20B is provided, two sheaths 201 and 202 each having a single lumen may be provided, as shown in Fig. 13, such that the sheaths 201 and 202 respectively accommodate the wires 10A and 10B therein.

With this configuration, the sheaths 201 and 202 covering the outer peripheral surfaces of the wires 10A and 10B can similarly prevent the wires 10A and 10B from coming into contact with each other when the rolling joint 8 rotates. In addition, the space occupied by the sheaths 201 and 202 can be reduced, as compared with the sheath 2. Furthermore, by providing the sheaths 201 and 202 individually for the respective wires 10A and 10B, the amount of twisting occurring in the individual sheaths 201 and 202 when the wires 10A and 10B twist becomes smaller than that in the sheath 2. Specifically, since the elasticity required in the sheaths 201 and 202 can be reduced, a material with low elasticity can be used for the sheaths 201 and 202.

### {Third Embodiment}

Next, a treatment device 101 and a surgical system 100 according to a third embodiment of the present invention will be described with reference to Fig. 14.

In the surgical system 100 shown in Fig. 14, the treatment device 101 according to this embodiment is inserted into a body via, for example, a treatment-device channel (not shown) provided in an insertion section 30a of an endoscope 30 so as to be used for treating, for example, an affected area.

As shown in Fig. 14, the surgical system 100 includes the endoscope 30 having the insertion section 30a to be inserted into a body; a manipulation input device 31 that is to be manipulated by a surgeon (operator) O and that outputs a manipulation command; a display unit 32 for displaying an image acquired by the endoscope 30; a driving device 33 that drives a bending section, which is provided at the distal end of the insertion section 30a, and the joints 7 and 8 of the treatment device 101; and a control device 34 that controls the driving device 33 in accordance with the manipulation command. For example, the insertion section 30a is inserted into the large intestine through the anus of a patient P lying on a surgical table 35.

The manipulation input device 31 has two manipulation arms 31A and 31B each having an articulated structure. The manipulation arm 31A is for bending the bending section of the insertion section 30a of the endoscope 30, and the manipulation arm 31B is for manipulating the joints 7 and 8 of the treatment device 101. The surgeon O manipulates the manipulation arm 31A to appropriately bend the bending section at the distal end of the insertion section 30a within the large intestine, so that the distal end surface protruding from the distal end of the treatment device 101 faces the affected area.

In this embodiment, the treatment device 101 differs from the treatment device 1 in the first and second embodiments described above in that the joints 7 and 8 are electrically driven by the driving device 33 instead of being driven by the manipulation unit 5 and the driving mechanism 6. Other components of the treatment device 101 are similar to those of the treatment device according to the first or second embodiment.

The driving device 33 has an electric motor (not shown). The electric motor pulls the proximal end of the wire 10A or 10B on the basis of a command signal from the control device 34, whereby the bendable joint 7 is driven. Furthermore, the driving device 33 also rotationally drives the rolling joint 8 on the basis of a command signal from the control device 34.

Next, the operation of the treatment device 101 and the surgical system 100 according to this embodiment, having the above-described configuration, will be described below.

In the surgical system 100, when a manipulation command is input to the control device 34 as a result of a manipulation performed on the manipulation arm 31B of the manipulation input device 31, the control device 34 actuates the driving device 33 of the treatment device 101, and the driving device 33 bends the bendable joint 7 or rotates the rolling joint 8 in accordance with the manipulation input to the manipulation arm 31B.

Accordingly, in this embodiment, the driving device 33 that generates a driving force for the joint 7 or 8 in response to an electrical command signal from the control device 34 is provided. This is advantageous in that the joint 7 or 8 can be driven by means of the manipulation input device 31 provided separately from the main unit 2 of the treatment device 101. Since other advantages of this embodiment are similar to those of the first and second embodiments, descriptions thereof will be omitted.

The articulated structures of the treatment devices 1 and 101 described in the first to third embodiments are merely examples and are modifiable, where appropriate. For example, the number of joints may be two or three or more. Furthermore, although the bendable joint 7 is described as a driven joint provided between the end effector 3 and the rolling joint 8, the driven joint may be a type of joint other than the bendable joint 7 and may be a joint provided in the end effector 3 (e.g., a joint for opening and closing grasping forceps).

Furthermore, although the wires 10A and 10B are described as examples of power transmission members in the first to third embodiments, the power transmission members are not limited to this type. For example, the power transmission members may be other linear components, such as rods, tubes, or metallic coils.

### {Reference Signs List}

- 1, 101: treatment device
- 2: main unit
- 3: end effector
- 4: joint unit
- 5: manipulation unit
- 6: driving mechanism
- 7: bendable joint (driven joint)
- 7a: pulley
- 7b: rotating shaft
- 8: rolling joint
- 9: housing
- 9a: proximal end
- 9b: inner wall
- 10A, 10B: wire (power transmission member)
- 11: pulling unit
- 12: shaft member
- 12a: groove
- 12b: hole
- 13: wire holder, through-hole (position maintaining means)
- 14: wire holder, channel (position maintaining means)
- 15: bearing
- 16: extension member
- 20, 201, 202: sheath
- 20A, 20B: lumen
- 30: endoscope
- 30a: insertion section
- 31: manipulation input device
- 32: display unit
- 33: driving device
- 34: control device
- 35: surgical table
- 100: surgical system

## Claims

1. A treatment device comprising:
a narrow main unit;
an end effector located at a distal end of the main unit;
a rolling joint that is provided between the end effector and the main unit and that is rotatable relative to the main unit about a longitudinal axis of the main unit;
a driven joint that is provided at the end effector or between the end effector and the rolling joint and that rotates together with the rolling joint about the longitudinal axis;
a plurality of linear power transmission members that extend parallel to each other from the driven joint to the main unit via the rolling joint and that transmit power applied to proximal ends;
a position maintaining means that maintains the plurality of power transmission members at predetermined relative positions, which are two positions located between the driven joint and the main unit and separated from each other by a certain distance in a direction of the longitudinal axis; and
a columnar shaft member disposed in the direction of the longitudinal axis between the two positions,
wherein the position maintaining means maintains the plurality of power transmission members at the relative positions located at a radially outer side of the shaft member and separated from each other by the certain distance in a circumferential direction of the shaft member.

2. The treatment device according to Claim 1,
wherein the shaft member is provided in a rotatable manner in the circumferential direction relative to the driven joint.

3. The treatment device according to Claim 1 or 2, further comprising:
a small-diameter extension member whose distal end is connected to the end effector and that extends from the end effector to the main unit,
wherein the shaft member has a hole extending therethrough in the direction of the longitudinal axis and accommodating a longitudinal section of the extension member.

4. The treatment device according to Claim 3,
wherein the shaft member is fixed to an outer peripheral surface of the extension member.

5. The treatment device according to any one of Claims 1 to 4,
wherein the shaft member has a groove that extends in the circumferential direction and that is located at a position where an outer peripheral surface of the shaft member comes into contact with the power transmission members when the rolling joint rotates.

6. The treatment device according to any one of Claims 1 to 5,
wherein the end effector is an energy device, grasping forceps, a local injection needle, a suction tube, or a clip.

7. A treatment device comprising:
a narrow main unit;
an end effector located at a distal end of the main unit;
a rolling joint that is provided between the end effector and the main unit and that is rotatable relative to the main unit about a longitudinal axis of the main unit;
a driven joint that is provided at the end effector or between the end effector and the rolling joint and that rotates together with the rolling joint about the longitudinal axis;
a plurality of linear power transmission members that extend parallel to each other from the driven joint to the main unit via the rolling joint and that transmit power applied to proximal ends; and
a tubular sheath that is composed of an elastic material and that covers outer peripheral surfaces of the plurality of power transmission members between the driven joint and the main unit.

8. The treatment device according to Claim 7,
wherein the sheath is formed of a single component having a plurality of lumens extending therethrough in a longitudinal direction, and
wherein the power transmission members are respectively inserted through the plurality of lumens.

9. The treatment device according to Claim 7,
wherein a plurality of the sheaths are provided individually for the respective power transmission members.

10. A surgical system comprising:
the treatment device according to any one of Claims 1 to 9;
a manipulation input device that is to be manipulated by an operator;
a driving device that drives at least one of the rolling joint and the driven joint; and
a control device that controls the driving device on the basis of a manipulation input to the manipulation input device.
